# EUROPEAN PATENT SPECIFICATION

(11) **EP 2 200 673 B1**
(45) Date of publication and mention of the grant of the patent: **21.12.2011**
(21) Application number: 08832765.5
(22) Date of filing: 19.09.2008
(51) Int. Cl.: A61L 31/10, A61L 31/16

(54) **MEDICAL DEVICES HAVING NANOFIBER-TEXTURED SURFACES**
MEDIZINISCHE VORRICHTUNGEN MIT OBERFLÄCHEN MIT NANOFASER-STRUKTUR
DISPOSITIFS MÉDICAUX À SURFACES TEXTURÉES EN NANOFIBRES

(30) Priority: 21.09.2007 US 994881 P
(43) Date of publication of application: 30.06.2010
(73) Proprietor: Boston Scientific Scimed, Inc., Maple Grove, MN 55311-1566 (US)
(72) Inventor: FLANAGAN, Aiden, Galway (IE)
(74) Representative: Vossius & Partner
(86) International application number: PCT/US2008/077112
(87) International publication number: WO 2009/039438

(56) References cited:
- WO-A2-2004/034931
- WO-A2-2005/039664
- WO-A2-2009/101472
- US-A1- 2005 038 498

## Description

### FIELD OF THE INVENTION

The present invention relates to medical devices and more particularly to medical devices having textured surfaces.

### BACKGROUND OF THE INVENTION

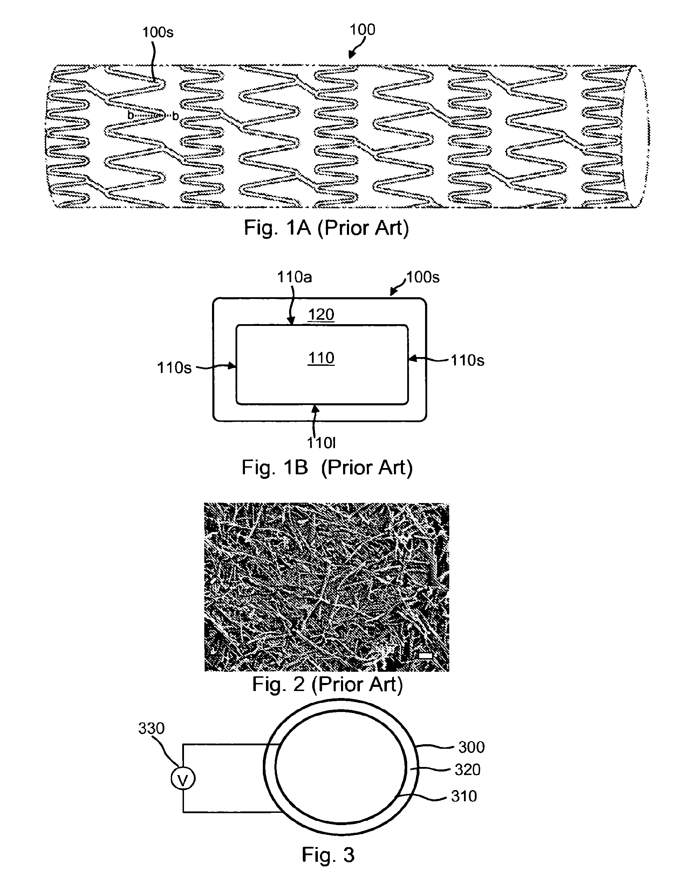

Coronary stents such as those commercially available from Boston Scientific Corp. (TAXUS and PROMUS), Johnson & Johnson (CYPHER), and others are frequently prescribed use for maintaining blood vessel patency. These products are based on metallic stents with biostable polymer coatings, which release antiproliferative therapeutic agents at a controlled rate and total dose, for preventing restenosis of the blood vessel. One such device is schematically illustrated, for example, in Figs. 1A and 1B. Fig. 1A is a schematic perspective view of a stent 100 which contains a number of interconnected struts 101. Fig. 1B is a cross-section taken along line b--b of strut 100s of stent 100 of Fig. 1A, and shows a stainless steel strut substrate 110 and a therapeutic-agent-containing coating 120, which encapsulates the entire stent strut substrate 110, covering the luminal 1101, abluminal 110a, and side 110s surfaces thereof.

It has been found that endothelialization of such implanted stents may be slow, and attached cells can be non-viable or non-functional. What is desired is a luminal surface that promotes relatively rapid formation of a functional endothelial cell layer, which is known to be effective for purposes of reducing or eliminating inflammation and thrombosis, which can occur in conjunction with the implantation of a foreign body in the vasculature. See, e.g., J. M. Caves et al., J. Vasc. Surg. (2006) 44: 1363-8.

US2005/038498 discloses a stent comprising a body structure having one or more surfaces having a plurality of nanostructured fibers.

WO2004/034931 discloses a stent assembly comprises an expandable tubular stent with an external surface of which is provided with a fabric which may constitute a reservoir to hold drugs to be released.

WO2005/039664 discloses a guide wire or an embolization device, or a guide shaft for a catheter comprising a solid and/or non-expandable core member made from e.g. metal and an outer surface layer, which is formed by electrospun nanofibers.

### SUMMARY OF THE INVENTION

According to the present invention, medical devices are provided which comprise (a) a substrate having first and second surfaces, (b) a nanofiber-textured layer comprising nanofibers disposed over at least the first surface of the substrate and defining a nanotextured outer surface for the device, and (c) a therapeutic-agent-eluting layer comprising a therapeutic agent disposed over at least the second surface of the substrate as disclosed in the claims.

### BRIEF DESCRIPTION OF THE DRAWINGS

Fig. 1A is a schematic perspective view of a stent in accordance with the prior art. Fig. 1B is a schematic cross-sectional view taken along line b-b of Fig. 1A.

Fig. 2 is an SEM image of compacted carbon nanofibers (scale bar=1 micron) in accordance with the prior art.

Fig. 3 is a schematic illustration of an electrochemical apparatus for electrodepositing nanofibers on the luminal surface of a stent (viewed along the axis of the stent), in accordance with an embodiment of the present invention.

Figs. 4A to 4C are schematic cross sectional views of stent struts, in accordance with various additional embodiments of the present invention.

### DETAILED DESCRIPTION OF THE INVENTION

According to an aspect of the present invention, medical devices are provided which comprise (a) a substrate having first and second surfaces, (b) a nanofiber-textured layer comprising nanofibers disposed over at least a portion of the first surface and defining a nanotextured outer surface for the device, and (c) a therapeutic-agent-eluting layer comprising a therapeutic agent disposed over at least a portion of the second surface. The nanofiber-textured layer may optionally comprise a further therapeutic agent.

In certain embodiments, the therapeutic-agent-eluting layer may overlie a portion of the nanofiber-textured layer, in which case the nanofiber-textured layer may improve adhesion for the therapeutic-agent-eluting layer.

In certain embodiments, the substrate may be a vascular stent, the first surface may be the luminal surface of the stent, the second surface may be the abluminal surface of the stent, and the therapeutic agent may be, for example, an agent that prevents or inhibits the proliferation of smooth muscle cells (an antiproliferative agent), an anti-inflammatory agent, or an agent that promotes the attachment and/or growth of endothelial cells, among many other possible agents. The nanofiber-textured layer may optionally comprise a further therapeutic agent, for example, an agent that promotes the attachment and/or growth of endothelial cells, an agent that inhibits growth of smooth muscle cells, or an anti-inflammatory agent, among others.

In these embodiments, the nanofiber-textured layer is disposed over at least a portion of the luminal surface of the stent and defines a nanotextured luminal surface for the stent (which promotes attachment and/or growth of endothelial cells), while the therapeutic-agent-eluting layer is disposed over at least a portion of the abluminal surface of the stent and affects release of a suitable therapeutic agent into a surrounding blood vessel. For example, an antiproliferative agent can be released, which prevents or inhibits smooth muscle cell growth. In this regard, an advantage of these embodiments of the invention is that therapeutic-agent-eluting stents may be provided, which prevent or inhibit restenosis like current state-of-the-art coated stents, but which also allow endothelium regeneration (healing) at a rate greater than such stents.

Moreover, in some of these embodiments, the stent comprises multiple stent struts, and a nanofiber-textured layer is disposed over the surfaces of the stent between the luminal and abluminal surfaces (e.g., over at least a portion of the side surfaces of the stent struts) thereby defining additional nanotextured outer surfaces for endothelial cell attachment and/or growth. In these embodiments, the therapeutic-agent-eluting layer typically does not cover a substantial portion of the nanofiber-textured layer on the side surfaces of the stent struts (e.g., no more than 25%, more preferably no more than 10%, even more preferably no more than 5% of the side surfaces), and preferably is absent from the side surfaces.

As indicated above, in some embodiments, the therapeutic-agent-eluting polymer coating may be disposed over a portion of the nanofiber-textured layer, in which case the nanofiber-textured layer may improve the adhesion of the therapeutic-agent-eluting coating. For example, the nanofiber-textured layer may be disposed on both the luminal and abluminal surfaces of a stent while the therapeutic-agent-eluting coating is disposed over only the abluminal surface of the stent (and on the nanofiber-textured layer). The therapeutic-agent-eluting coating may be sufficiently thick in such embodiments, however, such that the outer surface of the therapeutic-agent-eluting coating does not reflect the nanotextured surface of the underlying nanofiber-textured layer. Under such circumstances, care may be taken to ensure that little or none of the nanofiber-textured layer extending beyond the abluminal surface is covered by the therapeutic-agent-eluting coating.

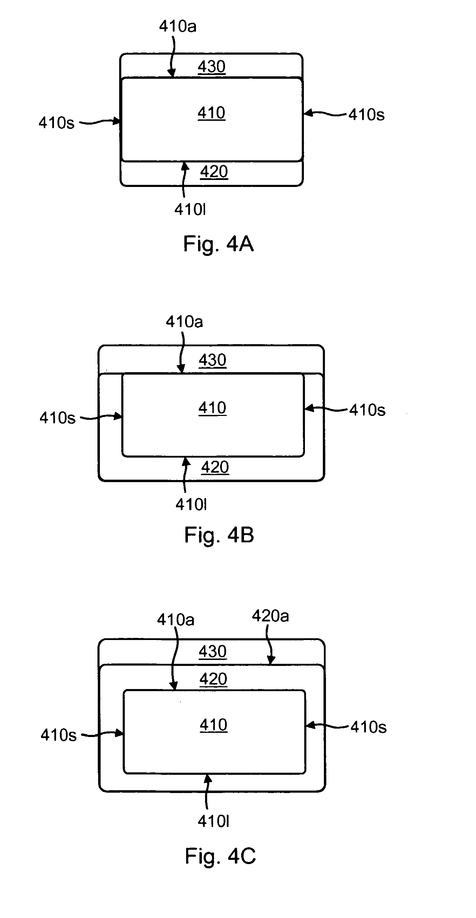

As a specific example of a stent in accordance with the invention, and with reference to the schematic cross sectional view of the stent strut 401 of Fig. 4A, a nanofiber-textured layer 420 may be provided over only the luminal surface 4101 of the stent strut substrate 410, but not the abluminal 410a and side 410s surfaces, whereas a drug-eluting layer 430 may be provided over the abluminal surface 410a of the stent strut substrate 410, but not the luminal 4101 and side 410s surfaces.

As another example, with reference to Fig. 4B, a nanofiber-textured layer 420 may be provided over the luminal 4101 and side 410s surfaces of the stent strut substrate 410, but not the abluminal surface 410a, whereas a drug-eluting layer 430 may again be provided over the abluminal surface 410a of the stent strut substrate 410, but not the luminal 4101 and side 410s surfaces.

As yet another example, with reference to Fig. 4C, a nanofiber-textured layer 420 may be provided over the luminal 4101, abluminal 410a and side 410s surfaces of the stent strut substrate 410, whereas the drug-eluting layer 430 may be again provided over the abluminal surface 420a of the nanofiber-textured layer 420, but not the luminal 4101 and side 410s surfaces.

Examples of suitable medical devices for use in the present invention vary widely and include implantable or insertable medical devices, for example, selected from the following: stents (including coronary vascular stents, peripheral vascular stents, cerebral, urethral, ureteral, biliary, tracheal, gastrointestinal and esophageal stents), stent coverings, stent grafts, vascular grafts, abdominal aortic aneurysm (AAA) devices (e.g., AAA stents, AAA grafts), vascular access ports, dialysis ports, catheters (e.g., urological catheters or vascular catheters such as balloon catheters and various central venous catheters), guide wires, balloons, filters (e.g., vena cava filters and mesh filters for distil protection devices), embolization devices including cerebral aneurysm filler coils (including Guglilmi detachable coils and metal coils), septal defect closure devices, myocardial plugs, patches, pacemakers, lead coatings including coatings for pacemaker leads, defibrillation leads, and coils, ventricular assist devices including left ventricular assist hearts and pumps, total artificial hearts, shunts, valves including heart valves and vascular valves, anastomosis clips and rings, cochlear implants, tissue bulking devices, and tissue engineering scaffolds for cartilage, bone, skin and other in vivo tissue regeneration, sutures, suture anchors, tissue staples and ligating clips at surgical sites, cannulae, metal wire ligatures, urethral slings, hernia "meshes", artificial ligaments, orthopedic prosthesis such as bone grafts, bone plates, fins and fusion devices, joint prostheses, orthopedic fixation devices such as interference screws in the ankle, knee, and hand areas, tacks for ligament attachment and meniscal repair, rods and pins for fracture fixation, screws and plates for craniomaxillofacial repair, dental implants, or other devices that are implanted or inserted into the body and from which therapeutic agent is released. The medical devices of the present invention include, for example, implantable and insertable medical devices that are used for systemic treatment, as well as those that are used for the localized treatment of any tissue or organ. As used herein, "treatment" refers to the prevention of a disease or condition, the reduction or elimination of symptoms associated with a disease or condition, or the substantial or complete elimination of a disease or condition.

As used herein a "layer" of a given material is a region of that material whose thickness is small compared to both its length and width. As used herein a layer need not be planar, for example, taking on the contours of an underlying substrate. A layer can be discontinuous (e.g., patterned). Terms such as "film,'' "layer" and "coating" may be used interchangeably herein.

As used herein, a "therapeutic-agent-eluting layer" is a layer that comprises a therapeutic agent and from which at least a portion of the therapeutic agent is eluted upon implantation or insertion into a subject. Subjects are vertebrate subjects, more typically mammalian subjects, and include human subjects, pets and livestock.

The therapeutic-agent-eluting layer will typically comprise, for example, from 1 wt% or less to 2 wt% to 5 wt% to 10 wt% to 25 wt% to 50 wt% to 75% to 90% to 95% to 98% to 99% or more of a single therapeutic agent or of a mixture of therapeutic agents within the layer. Therapeutic agents may be selected, for example, from those listed below, among others.

As used herein, a "therapeutic-agent-eluting polymeric layer" is a therapeutic-agent-eluting layer that further comprises a one or more polymers. The therapeutic-agent-eluting polymeric layer will typically comprise, for example, from 50 wt% or less to 75 wt% to 90 wt% to 95 wt% to 97.5 wt% to 99 wt% or more of a single polymer or a mixture differing polymers within the layer.

The polymer(s) within the therapeutic-agent-eluting polymeric layer may be biostable or biodisintegrable (i.e., materials that, upon placement in the body, are dissolved, degraded, resorbed, and/or otherwise removed from the placement site) and may be selected, for example, from one or more of the following: polycarboxylic acid polymers and copolymers including polyacrylic acids; acetal polymers and copolymers; acrylate and methacrylate polymers and copolymers (e.g., n-butyl methacrylate); cellulosic polymers and copolymers, including cellulose acetates, cellulose nitrates, cellulose propionates, cellulose acetate butyrates, cellophanes, rayons, rayon triacetates, and cellulose ethers such as carboxymethyl celluloses and hydroxyalkyl celluloses; polyoxymethylene polymers and copolymers; polyimide polymers and copolymers such as polyether block imides, polyamidimides, polyesterimides, and polyetherimides; polysulfone polymers and copolymers including polyarylsulfones and polyethersulfones; polyamide polymers and copolymers including nylon 6,6, nylon 12, polyether-block copolyamide polymers (e.g., Pebax® resins), polycaprolactams and polyacrylamides; resins including alkyd resins, phenolic resins, urea resins, melamine resins, epoxy resins, allyl resins and epoxide resins; polycarbonates; polyacrylonitriles; polyvinylpyrrolidones (cross-linked and otherwise); polymers and copolymers of vinyl monomers including polyvinyl alcohols, polyvinyl halides such as polyvinyl chlorides, ethylene-vinylacetate copolymers (EVA), polyvinylidene chlorides, polyvinyl ethers such as polyvinyl methyl ethers, vinyl aromatic polymers and copolymers such as polystyrenes, styrene-maleic anhydride copolymers, vinyl aromatic-hydrocarbon copolymers including styrenebutadiene copolymers, styrene-ethylene-butylene copolymers (e.g., a polystyrene-polyethylene/butylene-polystyrene (SEBS) copolymer, available as Kraton® G series polymers), styrene-isoprene copolymers (e.g., polystyrene-polyisoprene-polystyrene), acrylonitrile-styrene copolymers, acrylonitrile-butadiene-styrene copolymers, styrenebutadiene copolymers and styrene-isobutylene copolymers (e.g., polyisobutylene-polystyrene block copolymers such as SIBS), polyvinyl ketones, polyvinylcarbazoles, and polyvinyl esters such as polyvinyl acetates; polybenzimidazoles; ionomers; polyalkyl oxide polymers and copolymers including polyethylene oxides (PEO); polyesters including polyethylene terephthalates, polybutylene terephthalates and aliphatic polyesters such as polymers and copolymers of lactide (which includes lactic acid as well as d-,1- and meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (a copolymer of polylactic acid and polycaprolactone is one specific example); polyether polymers and copolymers including polyarylethers such as polyphenylene ethers, polyether ketones, polyether ether ketones; polyphenylene sulfides; polyisocyanates; polyolefin polymers and copolymers, including polyalkylenes such as polypropylenes, polyethylenes (low and high density, low and high molecular weight), polybutylenes (such as polybut-1-ene and polyisobutylene), polyolefin elastomers (e.g., santoprene), ethylene propylene diene monomer (EPDM) rubbers, poly-4-methyl-pen-1-enes, ethylene-alpha-olefin copolymers, ethylene-methyl methacrylate copolymers and ethylene-vinyl acetate copolymers; fluorinated polymers and copolymers, including polytetrafluoroethylenes (PTFE), poly(tetrafluoroethylene-co-hexafluoropropene) (FEP), modified ethylene-tetrafluoroethylene copolymers (ETFE), and polyvinylidene fluorides (PVDF); silicone polymers and copolymers; polyurethanes; p-xylylene polymers; polyiminocarbonates; copoly(ether-esters) such as polyethylene oxide-polylactic acid copolymers; polyphosphazincs; polyalkylene oxalates; polyoxaamides and polyoxaesters (including those containing amines and/or amido groups); polyorthoesters; biopolymers, such as polypeptides, proteins, and polysaccharides, including fibrin, fibrinogen, collagen, elastin, chitosan, gelatin, starch, and glycosaminoglycans such as hyaluronic acid; as well as blends and further copolymers of the above.

In some embodiments, the therapeutic-agent-eluting layer comprises one or more therapeutic agents and one or more one or more inorganic materials, for example, selected from carbon, metals and ceramic materials, among others. In these embodiments, the therapeutic-agent-eluting layer will typically comprise, for example, from 50 wt% or less to 75 wt% to 90 wt% to 95 wt% to 97.5 wt% to 99 wt% or more of a single inorganic material or a mixture of differing inorganic materials within the layer. The layer may be porous or non-porous. Specific examples of inorganic materials include pyrolytic carbon or other PVD carbon, porous metals and porous ceramics such as porous titanium oxide and porous aluminum oxide, among many others, including those listed below for use in nanofibers.

The thickness of the therapeutic-agent-eluting layer may vary widely, typically ranging from 10 nm to 25 nm to 50 nm to 100 nm to 250 nm to 500nm to 1 µm to 2.5 µm to 5 µm to 10 µm to 20 µm or more in thickness.

As used herein, a "nanofiber-textured layer" is a layer that comprises nanofibers, which nanofibers provide the layer with a surface texture.

The thickness of the nanofiber-textured layers of the invention may vary widely, typically ranging from 10 nm or less to 25 nm to 50 nm to 100 nm to 250 nm to 500nm to 1 µm to 2.5 µm to 5 µm to 10 µm to 20 µm or more in thickness.

The nanofiber-textured layer will typically comprise, for example, from 5 wt% or less to 10 wt% to 25 wt% to 50 wt% to 75 wt% to 90 wt% to 95 wt% or more of one or more types of nanofiber within the layer.

In some embodiments, the nanofiber-textured layer will optionally comprise a single polymer or a mixture of polymers. Such polymers may be selected, for example, from one or more of the polymers listed above for use in therapeutic-agent-eluting polymeric layers. In such embodiments, the nanofiber-textured layer will typically comprise, for example, from 5 wt% or less to 10 wt% to 25 wt% to 50 wt% to 75 wt% to 90 wt% to 95 wt% or more of a single polymer or a mixture polymers within the layer.

As used herein a "fiber" is a high aspect ratio particle, by which is meant that the length divided by the width (i.e., the largest cross sectional dimension taken perpendicular to the length, for instance, the diameter for a solid cylindrical or tubular filament, the width for a ribbon shaped filament, and so forth) is greater than 10, for example ranging from 10 to 25 to 50 to 100 to 250 to 500 to 1000 or more.

As used herein, a "nanofiber" is a fiber whose width is less than 1000 nm and preferably less than 100 nm, for example, ranging from 1000 nm to 500 nm to 250 nm to 100 nm to 50 nm to 25 nm to 10 nm or less. Nanofibers for use in the present invention may be formed from a variety of materials, as discussed below, and may be provided in a variety of sizes and shapes. For example, they may be solid or hollow, and they may be of regular or irregular geometry. Thus, nanofibers for use in the invention thus include tubular, solid cylindrical, and ribbon-shaped nanofibers, among many others.

Without wishing to be bound by theory, feature sizes less than 100 nm are believed to allow adhesion of proteins such as fibronectin, laminin, and/or vitronectin to the surface of the nanofiber-textured layer, and to provide a conformation for these proteins that better exposes amino acid sequences such as RGD and YGSIR which enhance endothelial cell binding. See, e.g., Standard handbook of biomedical engineering and design, Myer Kutz, Ed., 2003

ISBN 0-07-135637-1, p. 16.13. Moreover, nanotexturing increases surface energy, which is believed to increases cell adhesion. See, e.g., J.Y. Lim et al., J. Biomed. Mater. Res. (2004) 68A(3): 504-512. In this regard, submicron topography, including pores, fibers, and elevations in the sub-100 nm range, has been observed for the basement membrane of the aortic valve endothelium as well as for other basement membrane materials. See R.G. Flemming et al., Biomaterials 20 (1999) 573-588, S. Brody et al., Tissue Eng. 2006 Feb; 12(2): 413-421, and S.L. Goodman et al., Biomaterials 1996; 17: 2087-95. Goodman et al. employed polymer casting to replicate the topographical features of the subendothelial extracellular matrix surface of denuded and distended blood vessels, and they found that endothelial cells grown on such materials spread faster and appeared more like cells in their native arteries than did cells grown on untextured surfaces.

As noted above, in certain embodiments, medical devices in accordance with the invention are stents having luminal and abluminal surfaces. These embodiments are advantageous, for example, in that a therapeutic-agent eluting layer may be provided on the abluminal strut surfaces, for instance, a layer may be provided that elutes a therapeutic agent for the reduction or prevention of smooth muscle cell proliferation and restenosis. Nanotextured layers may be provided on the abluminal strut surfaces, and preferably the side surfaces of the struts as well, to encourage endothelial cell adhesion and the subsequent formation of a functional layer of endothelial cells. These nanotextured layers may further be provided with a therapeutic agent, for example, an agent that promotes endothelial cell attachment and/or growth. Formation of a functional endothelial cell layer is desirable for the reduction or elimination of inflammation and thrombosis. Upon implantation, the stent elutes an anti-restenosis agent into the vessel wall for a time period to inhibit or prevent restenosis, while simultaneously encouraging a functional endothelial layer to form on the blood-contacting surfaces of the stent. These embodiments are further advantageous in that cell-proliferation-inhibiting therapeutic agents will not be released in significant quantities from the luminal and side surfaces of the stent struts, where they may interfere with endothelial cell growth or where they may be released into the bloodstream, potentially causing undesirable side effects in locations removed from the stent.

As noted above, nanofibers for use in the present invention may be formed from a variety of materials, preferably inorganic materials (i.e., materials containing inorganic species, typically 50 wt% or more, for example, from 50 wt% to 75 wt% to 90 wt% to 95 wt% to 97.5 wt% to 99 wt% or more). Inorganic materials may be selected, for example, from suitable metallic materials (i.e., materials containing one or more metals, typically 50 wt% or more, for example, from 50 wt% to 75 wt% to 90 wt% to 95 wt% to 97.5 wt% to 99 wt% or more), which may be selected from the following: substantially pure metals including biostable metals such as gold, platinum, palladium, iridium, osmium, rhodium, titanium, tantalum, tungsten, and ruthenium, bioresorbable metals such as magnesium, zinc and iron, biostable metal alloys such as alloys comprising iron and chromium (e.g., stainless steels, including platinum-enriched radiopaque stainless steel), niobium alloys, titanium alloys including alloys comprising nickel and titanium (e.g., nitinol), alloys comprising cobalt and chromium, including alloys that comprise cobalt, chromium and iron (e.g., elgiloy alloys), alloys comprising nickel, cobalt and chromium (e.g., MP 35N) and alloys comprising cobalt, chromium, tungsten and nickel (e.g., L605), alloys comprising nickel and chromium (e.g., inconel alloys), and bioresorbable metal alloys such as magnesium alloys, zinc alloys and iron alloys (including their combinations with Ce, Ca, Zn, Mg, Fe, Zr and Li), and combinations of the foregoing, among many others. Inorganic materials may further be selected, for example, from suitable non-metallic inorganic materials (i.e., materials containing non-metallic inorganic materials, typically 50 wt% or more, for example, from 50 wt% to 75 wt% to 90 wt% to 95 wt% to 97.5 wt% to 99 wt% or more), including various metal- and non-metal-oxides (e.g., oxides of one or more of silicon, aluminum, titanium, zirconium, hafnium, tantalum, molybdenum, tungsten, rhenium, iron, niobium, and iridium), various metal- and non-metal-nitrides, various metal- and non-metal-carbides, various metal- and non-metal-borides, various metal- and non-metal-phosphates (e.g., calcium phosphate ceramics such as hydroxyapatite), various metal- and non-metal-sulfides, silicon and silicon-based ceramics such as those containing silicon nitrides, silicon carbides and silicon oxides (sometimes referred to as glass ceramics), carbon and carbon-based, ceramic-like materials such as carbon nitrides, and (c) hybrid materials (e.g., hybrid organic/inorganic materials, for instance, polymer/metallic-inorganic hybrids and polymer/non-metallic-inorganic hybrids).

Additional examples of nanofibers include magnetite nanofibers, silicate fibers such as aluminum silicate nanofibers and attapulgite clay, solid carbon nanofibers and carbon nanotubes. Specific examples of carbon nanotubes include single wall carbon nanotubes (SWNTs), which typically have outer diameters ranging from 0.25 nanometer to 5 nanometers, and lengths up to 10's of micrometers or more, and multi-wall carbon nanotubes (including so-called "few-wall" nanotubes), which typically have inner diameters ranging from 2.5 nanometers to 10 nanometers, outer diameters of 5 nanometers to 50 nanometers, and lengths up to 10's of micrometers or more, among others. A specific example of a suitable carbon nanofiber is a pyrolytically reduced carbon nanofiber available from Applied Sciences Inc. Increased cell adhesion has been observed on compacts of carbon nanofibers. See, e.g., B. Ercan et al., Technical Proceedings of the 2006 NSTI Nanotechnology Conference and Trade Show, Volume 2, pp. 127-128. Fig. 2 is an SEM image of a carbon nanofiber compact taken from this reference (scale bar=1 micron).

In addition to surface topography, cell adhesion and growth is also influenced by surface chemical properties. Thus, in ccrtain embodiments, the nanofibers, the optional polymer, or both, are provided with suitable chemical groups, for instance, in order to increase the surface energy of the nanofiber-textured layers or to otherwise promote cell adhesion.

For example, the nanofibers, the optional polymer, or both, may comprise hydroxyl, carboxyl, or other suitable functional groups to increase surface energy.

As another example, the nanofibers, the optional polymer, or both, may be provided with cell-adhesion-promoting polymers. More generally, cell-adhesion-promoting polymers may be associated with the nanofiber-textured layer in any suitable fashion, for example, covalently attached to the nanofibers, blended with the nanofibers (including encapsulation of the nanofibers), provided as one or more polymer blocks within a block copolymer, adsorbed to the surface of the nanofiber-textured layer, covalently attached to the surface of the nanofiber-textured layer, and so forth.

Cell-adhesion-promoting polymers include, for example, polymers consisting of or containing polypeptides containing cell-adhesive sequences, for example, antibodies that bind with endothelial cells and polymers which are known to promote endothelial cell adhesion by binding to integrins in the endothelial cell wall. For example, polypeptides containing RGD sequences (e.g., GRGDS) and WQPPRARI sequences are known to direct spreading and migrational properties of endothelial cells. See V. Gauvreau et al., Bioconjug Chem., 2005 Sep-Oct, 16(5), 1088-97. REDV tetrapeptide has been shown to support endothelial cell adhesion but not that of smooth muscle cells, fibroblasts, or platelets, and YIGSR pentapeptide has been shown to promote epithelial cell attachment, but not platelet adhesion. More information on REDV and YIGSR peptides can be found in U.S. Patent No. 6,156,572 and U.S. Patent Application No. 2003/008711 A further example of a cell-adhesive sequence is NGR tripeptide, which binds to CD 13 of endothelial cells. See, e.g., L. Holle et al., "In vitro targeted killing of human endothelial cells by co-incubation of human serum and NGR peptide conjugated human albumin protein bearing alpha (1-3) galactose epitopes," Oncol. Rep. 2004 Mar; 11(3):613-6.

Other polymers useful for cell adhesion may be selected from polymers consisting of or containing suitable members of the following, among others: the subunit chains found in collagen, laminin or fibroncctin, clastin chains, glycoprotein chains, polyanhydride chains, polyorthoester chains, polyphosphazene chains, and sulfated and non-sulfated polysaccharide chains, such as chitin, chitosan, sulfated and non-sulfated glycosaminoglycans as well as species containing the same such as proteoglycans, for instance, selected from heparin, heparin sulfate, chondroitin sulfates including chondroitin-4-sulfate and chondroitin-6-sulfate, hyaluronic acid, keratan sulfate, dermatan sulfate, hyaluronan, bamacan, perlecan, biglycan, fibromodulin, aggrecan, decorin, mucin, carrageenan, polymers and copolymers of uronic acids such as mannuronic acid, galatcuronic acid and guluronic acid, for example, alginic acid (a copolymer of beta-D-mannuronic acid and alpha-L-guluronic acid). See, e.g., U.S. Pat. App. No. 2005/0187146 to Helmus et al.

As indicated above, in certain embodiments, nanofibers for use in the present invention may be derivatized with various chemical entities. For example, the nanofibers may be covalently linked or "functionalized" with the chemical entities, or they may be otherwise associated with the chemical entities (e.g., by non-covalent interactions, encapsulation, etc.). Derivatization may result, for example, in improved processing (e.g., improved suspendability, improved interactions with an optional surrounding matrix material, etc.), improved cell adhesion, improved cell growth, as well as combinations of the forgoing, among other property improvements.

Although the discussion that follows is largely directed to techniques for functionalizing carbon nanofibers such as carbon nanotubes and solid nanofibers of carbon, analogous and non-analogous methods are also known for the functionalization of other nanofibers.

For example, in some embodiments of the invention, nanofibers are functionalized with simple organic and inorganic groups. For instance, the functionalization of carbon nanofibers with carboxyl, amino, halogen (e.g., fluoro), hydroxyl, isocyanate, acyl chloride, amido, ester, and O₃, functional groups, among others, has been reported. See, e.g., K. Balasubramanian and M. Burghard, "Chemically Functionalized Carbon Nanotubes," Small 2005, 1, No. 2, 180-192; T. Ramanathan et al., "Amino-Functionalized Carbon Nanotubes for Binding to Polymers and Biological Systems," Chem. Mater. 2005, 17, 1290-1295; C. Zhao et al., "Functionalized carbon nanotubes containing isocyanate groups," Journal of Solid State Chemistry, 177 (2004) 4394-4398; and S. Banerjee et al., "Covalent Surface Chemistry of Single-Walled Carbon Nanotubes," Adv. Mater. 2007, 17, No. 1, January 6, 17-29.

In some embodiments of the invention, nanofibers are functionalized with polymers. For example, polymer functionalized carbon nanofibers have been formed using so-called "grafting from" and "grafting to" approaches. In "grafting from" approaches, polymerization typically proceeds from an initiation site at the surface of the particle. "Grafting from" techniques typically involve (a) the attachment of polymerization initiators to the nanofibers surfaces, followed by (b) polymerization of monomers from the resulting particle-based macroinitiator. In the "grafting to" approach, pre-formed polymers are attached to particle surfaces. In a typical procedure, the preformed polymer has one or more reactive groups (e.g., reactive side groups or end groups) which may be directly reacted with functional groups on the nanofibers or which are linked to functional groups on the nanofibers by intermediate coupling species. An advantage of the "grafting to" approach is that it allows for the complete characterization and control of the polymers prior to grafting them to the nanofibers. In one specific example, N-protected amino acids have been linked to carbon nanotubes and subsequently used to attach peptides via fragment condensation or using a maleimido linker. See, e.g., S. Banerjee et al., "Covalent Surface Chemistry of Single-Walled Carbon Nanotubes," Adv. Mater. 2007, 17, No. 1, January 6, 17-29. Such techniques may be used, for example, to attach peptides which are known to promote endothelial cell adhesion, among others.

Substrate materials for the medical devices of the present invention may vary widely in composition and are not limited to any particular material, for example, being selected from biostable and biodisintegrable materials, organic and inorganic materials, and combinations of the foregoing. For example, substrate materials may be selected from (a) organic materials (i.e., materials containing organic species), for example, polymeric materials (i.e., materials containing polymers) such as those set forth above for use in therapeutic-agent-eluting polymeric layers, (b) inorganic materials (i.e., materials containing inorganic species) including metallic inorganic materials (i.e., materials containing metals) and non-metallic inorganic materials (i.e., materials containing non-metallic inorganic materials) such as those set forth above for use in nanofibers, and (c) hybrid materials (e.g., hybrid organic/inorganic materials, for instance, polymer/metallic-inorganic hybrids and polymer/non-mctallic-inorganic hybrids).

Various processes for forming medical devices in accordance with the invention will now be described. For example, in one embodiment, a suspension containing one or more types of nanofibers, one or more polymers, and one or more solvent species is contacted with the surface of a medical device substrate (e.g., a stent). The solvent species may include water, organic solvents, and mixtures thereof, and may be selected, for example, based on the ability of the solvent species to suspend the nanofibers, to dissolve the polymers, and so forth. If desired, other optional agents may be added, for example, one or more surfactants to aid in suspension of the nanofibers or one or more therapeutic agents, among others. The suspension may be contacted with the substrate using any suitable technique, including application to the substrate using a suitable application device such as a brush, roller, stamp or ink jet printer, by dipping the substrate, by spray coating the substrate using spray techniques including ultrasonic spray coating and electrohydrodynamic coating, among other methods. After application, the solvent is removed actively (e.g., by applying heat and/or vacuum) or passively (e.g., by allowing evaporation to occur), leaving a polymer coating on the substrate. Nanofibers entrapped in the polymer coating near the surface of the coating provide the coating with a nanotextured surface. In certain embodiments, the thin layer of polymer that covers the nanofibers at the surface of the polymer coating is removed, for example, by etching or ablation (e.g., using a UV laser to ablate a thin layer of the polymer surface), selectively exposing the upper surfaces of the nanofibers.

In another embodiment, a solution containing one or more polymers, one or more other optional agents, and one or more solvent species is contacted with the surface of a medical device substrate. The solvent species may include water, organic solvents, and mixtures thereof, and may be selected, for example, based on the ability of the solvent species to dissolve the polymers, among other factors. The solution may be contacted with the substrate using any suitable technique, for example, selected from those described above, and dried to form a polymeric layer. Subsequently, a suspension containing dispersed nanofibers and one or more solvent species capable of dissolving the one or more previously applied polymers is contacted with the polymeric layer using any suitable technique, for example, selected from those described above. When the solvent species come into contact with the polymeric layer, a surface region of the polymeric layer is dissolved by the solvent species, allowing the nanofibers to be at least partially submerged in the dissolved polymeric layer. The polymeric layer solidifies upon evaporation of the solvent species, adhering the nanofibers to the surface of the polymeric layer. By variation of the coating process parameters such as spray time and fluid flow, the amount of exposed bare surface of the nanoparticles can be modified.

In yet another embodiment, electrodeposition processes are used to deposit a layer of nanofibers on a conductive substrate (e.g., substrate having a metallic surface, a conductive metal oxide surface; a conductive polymer surface, etc.), for example, based on processes like those described in A.R. Boccaccini et al., "Electrophoretic deposition of carbon nanotubes," Carbon 44 (2006) 3149-3160. Briefly, the conductive substrate and a counter-electrode are immersed in a suspension of containing one or more solvent species, charged nanofibers (e.g., functionalized nanofibers, nanofibers modified with bound charged surfactant, etc.), and optional agents such as surfactants, salts, etc. A voltage is then applied across the electrodes, causing deposition to occur, with the migration direction for the nanofibers being controlled by surface charge. For example, oxidized carbon nanotubes are typically negatively charged and are attracted to the positive electrode (anode). In some embodiments, an electrochemically polymerizable monomer (e.g., pyrrole) may be added to the suspension, yielding a nanofiber-polymer composite layer.

Fig. 3 is a schematic illustration of an electrochemical apparatus for electrodepositing nanofibers on at least the luminal surface of a stent 300 (end view) in accordance with an embodiment of the invention. A nanofiber-containing suspension 320 is placed between the stent 300 and cylindrical counterelectrode 310 (end view). Nanofibers are deposited from the suspension 320 onto the stent 300 upon application of an appropriate voltage (using a suitable voltage source V) for an appropriate time.

In a blood vessel, the endothelial cells (which are elongated) align themselves with the blood flow. See, e.g., V. Fuster et al., Hurst's The Heart, 11th Ed., 2004, Chapter 7, Biology of the Vessel Wall, pp. 135 *et seq.* Therefore, in various vascular applications, including vascular stents, it may be desirable encourage this alignment on the device surface (e.g., on the luminal surface of a vascular stent parallel to the stent axis). Aligning the nanofibers in the direction of blood flow encourages the endothelial cells to also align themselves in the same direction. In this regard, certain particles, including carbon nanotubes and carbon nanofibers, are known to become aligned relative to an electric field. See, e.g., US 2006 0 368 738 entitled "Medical devices having electrically aligned nanofibers," U.S. Patent No. 6,837,928 entitled "Electric field orientation of carbon nanotubes," and X. Liu et al., "Electric-field oriented carbon nanotubes in different dielectric solvents," Current Applied Physics 4 (2004) 125-128. For example, in the present invention, the nanofibers may be aligned during the above-described processing (e.g., during nanofiber spray deposition, electrodeposition, etc.). For a cylindrical article such as a stent, the nanofibers may be aligned within a liquid suspension (e.g., within a suspension that has been sprayed on the device surface prior to solvent removal, or within a suspension adjacent the device surface during electrodeposition) along the length of the device by applying a suitable voltage across ring shaped electrodes placed near each end of the stent as described in US 2006 0 368 738. Alternatively a strong magnetic field may be used to align the nanofibers. The nanofibers may also be aligned by passing an electric current through a liquid suspension; this can be achieved, for example, by direct contact or by rotating the stent in a magnetic field during spraying, which induces a current perpendicular to the field.

Whether or not the elongated particles exhibit a degree of alignment in a certain direction can be determined, for example, by microscopic analysis of the particle-containing regions (e.g., using transmission electron microscopy). In some instances, particle alignment can be inferred from significant anisotropy in electrical, mechanical or other physical measurements, for example, exhibiting directional differences of 20% to 50% to 100% or more.

Thus, the above techniques can be used to form nanofiber-textured layers either with or without an accompanying polymeric material. Moreover, where a polymeric material is present, the nanofibers may be partially or completely covered by the polymeric material (e.g., completely or partially embedded in a polymeric matrix). Where a layer of polymeric material covers the nanofibers at the surface, the nanofibers may be exposed, for example, using etching or ablation techniques as described above. The nanofibers within the nanofiber-textured layers may either be aligned or non-aligned.

Therapeutic-agent-eluting layers may be disposed over a substrate (or over an underlying portion of a nanofiber-textured layer) using any suitable method known in the art. For example, where the layer contains one or more polymers having thermoplastic characteristics, the layer may be formed, for instance, by (a) providing a melt that contains polymer(s), therapeutic agent(s), and any other optional species desired and (b) subsequently cooling the melt. As another example, a layer may be formed, for instance, by (a) providing a solution or dispersion that contains one or more solvent species, therapeutic agent(s), and any other optional species desired, including optional polymer(s) or other optional non-polymeric matrix material(s), and (b) subsequently removing the solvent species. The melt, solution or dispersion may be disposed on a substrate surface, for example, by roll-coating the substrate (e.g., where it is desired to apply the layer to the abluminal surface of a tubular device such as a stent), by application to the substrate using a suitable application device such as a brush, roller, stamp or ink jet printer, by dipping the substrate, by spray coating the substrate using spray techniques such as ultrasonic spray coating and electrohydrodynamic coating, among other methods. In certain embodiments (e.g., dipping, spraying, etc.), a portion of the substrate is masked to prevent the therapeutic-agent-eluting layer from being applied thereon.

A wide variety of therapeutic agents may be employed in conjunction with the present invention, including genetic therapeutic agents, non-genetic therapeutic agents and cells, which may be used for the treatment of a wide variety of diseases and conditions. Numerous therapeutic agents are described here.

Suitable therapeutic agents for use in connection with the present invention may be selected, for example, from one or more of the following: (a) anti-thrombotic agents such as heparin, heparin derivatives, urokinase, clopidogrel, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promoters; (g) vascular cell growth inhibitors such as growth factor inhibitors, growth factor receptor antagonists, transcriptional repressers, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins; genistein, quinoxalines); (i) prostacyclin analogs; (j) cholesterol-lowering agents; (k) angiopoietins; (1) antimicrobial agents such as triclosan, cephalosporins, antimicrobial peptides such as magainins, aminoglycosides and nitrofurantoin; (m) cytotoxic agents, cytostatic agents and cell proliferation affectors; (n) vasodilating agents; (o)agents that interfere with endogenous vasoactive mechanisms, (p) inhibitors of leukocyte recruitment, such as monoclonal antibodies, (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) beta-blockers, (u) bARKct inhibitors, (v) phospholamban inhibitors, (w) Serca 2 gene/protein, (x) immune response modifiers including aminoquizolines, for instance, imidazoquinolines such as resiquimod and imiquimod, (y) human apolioproteins (e.g., AI, AII, AIII, AIV, AV, etc.).

Preferred therapeutic agents include paclitaxel (including particulate forms thereof, for instance, protein-bound paclitaxel particles such as albumin-bound paclitaxel nanoparticles, e.g., ABRAXANE), sirolimus, everolimus, tacrolimus, Epo D, dexamethasone, estradiol, halofuginone, cilostazole, geldanamycin, ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2), as well a derivatives of the forgoing, among others.

Numerous therapeutic agents, not necessarily exclusive of those listed above, have been identified as candidates for vascular and other treatment regimens, for example, as agents targeting restenosis. Such agents are useful for the practice of the present invention and suitable examples may be selected from one or more of the following: (a) Ca-channel blockers including benzothiazapines such as diltiazem and clentiazem, dihydropyridines such as nifedipine, amlodipine and nicardapine, and phenylalkylamines such as verapamil, (b) scrotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) Angiotensin Converting Enzyme (ACE) inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopcptin and ocrcotidc, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate, nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF, agents containing RGD and other peptide sequences that promote attachment of endothelial cells, antibodies responsive to endothelial cells, and (cc) blood rheology modulators such as pentoxifylline.

Numerous additional therapeutic agents useful for the practice of the present invention are also disclosed in U.S. Patent No. 5,733,925 to Kunz et al.

## Claims

1. An implantable or insertable medical device comprising (a) a substrate comprising first and second surfaces, (b) a nanofiber-textured layer comprising nanofibers, said nanofiber-textured layer disposed over at least the first surface of the substrate and defining a nanotextured surface for the medical device, and (c) a therapeutic-agent-eluting layer comprising a therapeutic agent disposed over at least the second surface of the substrate, wherein the the substrate is a vascular stent that comprises luminal and abluminal surfaces, wherein the nanofiber-textured layer is disposed over at least a portion of the luminal surface, wherein the therapeutic-agent-eluting layer is disposed over at least a portion of the abluminal surface but is not disposed over the luminal surface, and wherein the therapeutic agent is an agent that prevents or inhibits the proliferation of smooth muscle cells.

2. The medical device of claim 1, wherein nanofibers within at least that portion of the nanofiber-textured layer that is disposed over the luminal surface demonstrate a degree of alignment with a longitudinal axis of the stent.

3. The medical device of claim 1, wherein the stent comprises a plurality of stent struts, and wherein the nanofiber-textured layer is further disposed over at least a portion of the side surfaces of the stent struts and defines a nanotextured outer surface for at least a portion of the side surfaces of the stent struts, wherein the nanofiber-textured layer is optionally further disposed over at least a portion of the abluminal surface of the stent and wherein the therapeutic-agent-eluting layer covers a portion of the nanofiber-textured layer.

4. The medical device of claim 1, wherein the nanofiber-textured layer further comprises an agent that promotes the formation of an endothelial cell layer.

5. The medical device of claim 1, wherein the therapeutic-agent-eluting layer further comprises a biostable polymer and/or a biodisintegrable polymer.

6. The medical device of claim 1, wherein the therapeutic-agent-eluting layer covers a portion of the nanofiber-textured layer.

7. The medical device of claim 1, wherein the nanofiber-textured layer comprises carbon nanofibers selected from carbon nanofibers of solid cross-section, carbon nanofibers of hollow cross-section, and combinations thereof

8. The medical device of claim 1, wherein the nanofiber-textured layer comprises nanofibers having a width of less than 100 nm.

9. The medical device of claim 1, wherein the substrate is a metallic substrate.

10. The medical device of claim 1, wherein the nanofiber-textured layer further comprises a polymer, wherein said nanofibers are preferably completely embedded in a polymeric matrix or wherein said nanofibers are preferably partially exposed and partially embedded in a polymeric matrix.

11. The medical device of claim 10, wherein said nanofibers are partially exposed as a result of an ablation or etching step.

12. The medical device of claim 1, wherein the nanofiber-textured layer does not comprise a polymer.

13. The medical device of claim 1, wherein the nanofiber-textured layer comprises surface groups selected from surface hydroxyl groups, surface carboxyl groups, surface peptide groups, and combinations thereof, wherein the surface peptide groups preferably comprise cell-adhesive amino acid sequences.

14. The medical device of claim 13, wherein the nanofiber-textured layer comprises nanofibers derivatized with said surface groups.

15. The medical device of claim 1, wherein the therapeutic agent is selected from taxanes, rapamycin and rapamycin analogs.

## Patentansprüche

1. Implantierbare oder einführbare medizinische Vorrichtung, die aufweist: (a) ein Substrat, das erste und zweite Oberflächen aufweist, (b) eine nanofasertexturierte Schicht, die Nanofasern aufweist, wobei die nanofasertexturierte Schicht über mindestens der ersten Oberfläche des Substrats angeordnet ist und eine nanotexturierte Oberfläche für die medizinische Vorrichtung definiert, und (c) eine ein Therapeutikum eluierende Schicht, die ein Therapeutikum aufweist, das über mindestens der zweiten Oberfläche des Substrats angeordnet ist,
wobei das Substrat ein vaskulärer Stent ist, der luminale und abluminale Oberflächen aufweist;
wobei die nanofasertexturierte Schicht über mindestens einen Abschnitt der luminalen Oberfläche angeordnet ist, wobei die ein Therapeutikum eluierende Schicht über mindestens einen Abschnitt der abluminalen Oberfläche angeordnet ist, jedoch nicht über der luminalen Oberfläche angeordnet ist, und wobei das Therapeutikum ein Mittel ist, das die Proliferation glatter Muskelzellen verhindert oder hemmt.

2. Medizinische Vorrichtung nach Anspruch 1, wobei Nanofasern innerhalb mindestens jenes Abschnitts der nanofasertexturierten Schicht, die über der luminalen Oberfläche angeordnet sind, ein Maß an Ausrichtung mit einer Längsachse des Stents aufweisen.

3. Medizinische Vorrichtung nach Anspruch 1, wobei der Stent mehrere Stent-Streben aufweist, und wobei die nanofasertexturierte Schicht ferner über mindestens einen Abschnitt der Seitenflächen der Stent-Streben angeordnet ist und für mindestens einen Abschnitt der Seitenflächen der Stent-Streben eine nanotexturierte Außenfläche definiert,
wobei die nanofasertexturierte Schicht optional ferner über mindestens einen Abschnitt der abluminalen Oberfläche des Stents angeordnet ist und wobei die ein Therapeutikum eluierende Schicht einen Abschnitt der nanofasertexturierten Schicht bedeckt.

4. Medizinische Vorrichtung nach Anspruch 1, wobei die nanofasertexturierte Schicht ferner ein Mittel aufweist, das die Bildung einer Endothelzellschicht fördert.

5. Medizinische Vorrichtung nach Anspruch 1, wobei die ein Therapeutikum eluierende Schicht ferner ein biostabiles Polymer und/oder ein biologisch abbaubares Polymer aufweist.

6. Medizinische Vorrichtung nach Anspruch 1, wobei die ein Therapeutikum eluierende Schicht einen Abschnitt der nanofasertexturierten Schicht bedeckt.

7. Medizinische Vorrichtung nach Anspruch 1, wobei die nanofasertexturierte Schicht Kohlenstoffnanofasern aufweist, die aus Kohlenstoffnanofasern mit massivem Querschnitt, Kohlenstoffnanofasern mit hohlem Querschnitt und Kombinationen davon ausgewählt sind.

8. Medizinische Vorrichtung nach Anspruch 1, wobei die nanofasertexturierte Schicht Nanofasern mit einer Breite von weniger als 100 nm aufweist.

9. Medizinische Vorrichtung nach Anspruch 1, wobei das Substrat ein Metallsubstrat ist.

10. Medizinische Vorrichtung nach Anspruch 1, wobei die nanofasertexturierte Schicht ferner ein Polymer aufweist,
wobei die Nanofasern vorzugsweise vollständig in eine Polymermatrix eingebettet sind, oder
wobei die Nanofasern vorzugsweise teilweise freiliegen und teilweise in eine Polymermatrix eingebettet sind.

11. Medizinische Vorrichtung nach Anspruch 10, wobei die Nanofasern infolge eines Ablations- oder Ätzschritts teilweise freiliegen.

12. Medizinische Vorrichtung nach Anspruch 1, wobei die nanofasertexturierte Schicht kein Polymer aufweist.

13. Medizinische Vorrichtung nach Anspruch 1, wobei die nanofasertexturierte Schicht Oberflächengruppen aufweist, die aus Oberflächenhydroxylgruppen, Oberflächencarboxylgruppen, Oberflächenpeptidgruppen und Kombinationen davon ausgewählt sind,
wobei die Oberflächenpeptidgruppen vorzugsweise zellklebende Aminosäuresequenzen aufweisen.

14. Medizinische Vorrichtung nach Anspruch 13, wobei die nanofasertexturierte Schicht Nanofasern aufweist, die mit den Oberflächengruppen derivatisiert sind.

15. Medizinische Vorrichtung nach Anspruch 1, wobei das Therapeutikum aus Taxanen, Rapamycin und Rapamycin-Analoga ausgewählt ist.

## Revendications

1. Dispositif médical implantable ou insérable comprenant (a) un substrat comportant une première et une deuxième surfaces, (b) une couche texturée en nanofibres comprenant des nanofibres, ladite couche texturée en nanofibres étant disposée sur au moins la première surface du substrat et définissant une surface nanotexturée pour le dispositif médical, et (c) une couche d'élution d'agent thérapeutique comprenant a agent thérapeutique disposé sur au moins la deuxième surface du substrat,
où le substrat est une endoprothèse vasculaire qui comporte une surface luminale et une surface abluminale ;
où la couche texturée en nanofibres est disposée sur au moins une partie de la surface luminale, où la couche d'élution d'agent thérapeutique est disposée sur au moins une partie de la surface abluminale, mais n'est pas disposée sur la surface luminale, et où l'agent thérapeutique est un agent qui prévient ou inhibe la prolifération de cellules musculaires lisses.

2. Dispositif médical selon la revendication 1, où, à l'intérieur d'au moins la partie de la couche texturée en nanofibres qui est disposée sur la surface luminale, les nanofibres présentent un degré d'alignement avec un axe longitudinal de l'endoprothèse.

3. Dispositif médical selon la revendication 1, où l'endoprothèse comprend une pluralité d'entretoises d'endoprothèse, et où la couche texturée en nanofibres est en outre disposée sur au moins une partie des surfaces latérales des entretoises d'endoprothèse et définit une surface externe nanotexturée pour au moins une partie des surfaces latérales des entretoises d'endoprothèse,
où la couche texturée en nanofibres est en outre facultativement disposée sur au moins une partie de la surface abluminale de l'endoprothèse, et où la couche d'élution d'agent thérapeutique recouvre une partie de la couche texturée en nanofibres.

4. Dispositif médical selon la revendication 1, où la couche texturée en nanofibres comprend en outre un agent favorisant la formation d'une couche cellulaire endothéliale.

5. Dispositif médical selon la revendication 1, où la couche d'élution d'agent thérapeutique comprend en outre un polymère biostable et/ou un polymère biodégradable.

6. Dispositif médical selon la revendication 1, où la couche d'élution d'agent thérapeutique recouvre une partie de la couche texturée en nanofibres.

7. Dispositif médical selon la revendication 1, où la couche texturée en nanofibres comprend des nanofibres de carbone choisies parmi des nanofibres de carbone à section transversale pleine, des nanofibres de carbone à section transversale creuse, et des combinaisons de celles-ci.

8. Dispositif médical selon la revendication 1, où la couche texturée en nanofibres comprend des nanofibres de largeur inférieure à 100 nm.

9. Dispositif médical selon la revendication 1, où le substrat est un substrat métallique.

10. Dispositif médical selon la revendication 1, où la couche texturée en nanofibres comprend en outre un polymère,
les nanofibres étant de préférence totalement noyées dans une matrice polymère, ou
les nanofibres étant de préférence partiellement exposées et partiellement noyées dans une matrice polymère.

11. Dispositif médical selon la revendication 10, où les nanofibres sont partiellement exposées à la suite d'une étape d'extraction ou de gravure.

12. Dispositif médical selon la revendication 1, où la couche texturée en nanofibres ne comprend pas un polymère.

13. Dispositif médical selon la revendication 1, où la couche texturée en nanofibres comprend des groupes de surface choisis parmi des groupes de surface hydroxyles, des groupes de surface carboxyles, des groupes de surface peptides, et des combinaisons de ceux-ci,
les groupes de surface peptides comprenant préférentiellement des séquences amino-acides à adhérence cellulaire.

14. Dispositif médical selon la revendication 13, où la couche texturée en nanofibres comprend des nanofibres dérivatisées avec les groupes de surface.

15. Dispositif médical selon la revendication 1, où l'agent thérapeutique est choisi parmi les taxanes, la rapamycine et des agents analogues à la rapamycine.
